# EUROPEAN PATENT APPLICATION

(11) **EP 4 674 972 A1**
(43) Date of publication of application: **07.01.2026**
(21) Application number: 24306127.2
(22) Date of filing: 05.07.2024
(51) Int. Cl.: C12P 7/6463, C11C 3/00, C12N 1/00, C12N 1/12, C12M 1/00

(54) **METHOD OF PRODUCING A FATTY ESTER FROM MICROALGAE**

(71) Applicant: Institut National de la Recherche pour l'Agriculture, l'Alimentation et l'Environnement, 75007 Paris (FR); UNIVERSITE DE MONTPELLIER, 34090 Montpellier (FR); Institut national d'enseignement supérieur pour l'agriculture, l'alimentation et l'environnement, 75116 Paris (FR)
(72) Inventor: SUBILEAU, Maeva, 34070 Montpellier (FR); VAN LIS, Robert, 34200 Sète (FR); GROUSSEAU, Estelle, 34090 Montpellier (FR); DUBREUCQ, Eric, 34980 Saint Gély du Fesc (FR); RASAMISON, Annie, 34070 Montpellier (FR); RONCERAY, Alexis, 34300 AGDE (FR); MAHIEUX, Margot, 11100 Narbonne (FR); LACROUX, Julien, 31300 Toulouse (FR); TRABLY, Eric, 11590 Cuxac d`Aude (FR); STEYER, Jean-Philippe, 11100 Narbonne (FR)
(74) Representative: Santarelli

(57) **Abstract**

The invention relates to a method of producing an ester of a fatty acid and a C₁-C₄ alcohol, comprising successive steps of: cultivating cells of a microalga strain on a growth medium, harvesting the cultivated cells and concentrating them to obtain a wet cell pellet, suspending said wet cell pellet in an hydroalcoholic solution comprising said C₁-C₄ alcohol, incubating the cell suspension thus formed so as to form said ester of a fatty acid and a C₁-C₄ alcohol by transesterification and/or esterification of lipids produced by the microalga cells catalyzed by endogenous enzymes of said cells, and recovering said ester of a fatty acid and a C₁-C₄ alcohol from said cell suspension. The growth medium can advantageously be a dark fermentation effluent.

## Description

The invention lies in the field of the production of molecules of interest by renewable biomass.

More particularly, the invention relates to a method of producing a fatty ester from a microalga, in particular comprising cultivating said microalga on a dark fermentation effluent. Another object of the invention is a device for producing a fatty ester adapted for implementing such a method.

One of the major challenges in biotechnology research today is to provide solutions and alternatives for the preparation of bio-sourced molecules with low added value, which can be used for energy production and/or as building blocks in organic chemistry. In this context, biotransformation processes can enable innovations that are part of virtuous and sustainable cycles of valorization of biomass co-products.

To this end, processes using bacteria, such as dark fermentation or anaerobic digestion, are the most widespread, but microalgae can also be used for producing molecules of interest. Microalgae are a group of photosynthetic eukaryotic organisms which are found in various aquatic habitats. It has been observed by the prior art that they are in particular capable of accumulating lipids, which is of great interest for the field of oleochemistry, for example for the production of lipid-based biofuels (Kong et al., 2018, New Phytol. 218, 1340-1348). Microalgal lipid catabolism comprises in particular lipolysis, which is catalyzed by endogenous lipases of the microorganism and which releases fatty acids from glycerolipids, mostly polar glycolipids, contained in the cell membranes.

At present, production of lipid-based products by microalgae is not yet economically viable, and major technological bottlenecks remain in the cultivation and lipid extraction stages, as described for example in Araújo et al., 2021, Frontiers in Marine Science 7, 626389.

The present invention aims to propose a method for obtaining lipid derivatives, in particular fatty esters, from microalgae, which comprises as few steps as possible, which can be carried out at low cost and with a high productivity, and which can be easily implemented on an industrial scale.

An additional objective of the invention is for this method to use renewable resources, and, more preferably, waste, as substrates for the microalgae growth.

The inventors have now demonstrated the feasibility of an integrated process for the production of lipid derivatives, in particular esters of fatty acids, via endogenous enzymes present in microalgae. This process may use, as a microalgae culture medium, a medium comprising specific organic substances. In particular, it may use an effluent of the dark fermentation of food waste comprising these specific organic substances, and it can advantageously comprise cultivating the microalgae directly on such an effluent without pretreatment thereof.

Moreover, it has been discovered by the inventors that microalgae are able to produce fatty esters via enzymatic conversion of lipids within wet microalgal biomass, by simple addition of alcohol in the medium, without the addition of expensive exogenous enzymes, in particular lipases. In the presence of alcohol in an aqueous medium containing freshly harvested wet microalgal biomass, there occurs an *in situ* transformation of microalgal lipids, mainly glycerolipids (essentially polar membrane lipids), by the endogenous enzymes, in particular lipases, of the microalgae, which results in their conversion into fatty esters and free fatty acids with a high productivity, for example as high as 0.4 g/l of fatty esters and free fatty acids for the species *Ochromonas danica* in the presence of ethanol. A high ratio of fatty esters can furthermore be obtained with respect to the total content of fatty acyl chains that can be recovered at the end of the process. This ratio can even be higher than 90 % under the most favorable operating conditions, for example by implementing the species *Chlorella vulgaris* in the presence of ethanol. This conversion of microalgal lipids by the endogenous enzymes of the microalgae, into fatty esters and free fatty acids, with a high productivity, has been observed for microalgae from phylogenetically very distinct and diverse families, in particular *Chlorella sorokiniana*, *Chlorella vulgaris*, *Desmodesmus armatus*, *Euglena gracilis* var. bacillaris/saccharophila, *Ochromonas danica* and the marine alga *Phaeodactylum tricomutum*, therefore indicating that the metabolic capacities involved in the method of the invention are endemic to all microalgae.

Therefore, a first object of the invention is a method of producing one or several ester(s) of a fatty acid and a C₁-C₄, preferably C₁-C₂, alcohol, using microalgae.

This method comprises successive steps of:
- cultivating cells of a microalga strain on a suitable growth medium in suitable conditions to promote cell growth, and preferably also to promote lipid production by the cells,
- harvesting the cultivated cells and concentrating them to obtain a wet cell pellet,
- suspending this wet cell pellet in an hydroalcoholic solution comprising said C₁-C₄ alcohol,
- incubating the cell suspension thus formed, under stirring, at a temperature between 10 and 60 °C for 30 minutes to 48 hours, so as to form said ester(s) of a fatty acid and the C₁-C₄ alcohol by esterification and/or transesterification of lipids produced by the microalga cells, this esterification and/or transesterification being catalyzed by endogenous enzymes of these cells, this incubating step not implementing any exogenous catalyst of esterification and/or transesterification reactions,
- and recovering said ester(s) of a fatty acid and said C₁-C₄ alcohol from this cell suspension.

By endogenous enzymes, it is herein meant the enzymes produced by the microalga strain implemented in the method of the invention. These enzymes may be wild-type enzymes and/or result from any form of genetic modification of the strain. By exogenous catalyst, it is meant any enzyme or other chemical substance capable of catalyzing a transesterification and/or esterification reaction, which is not produced by the microalga or by any other microorganism that could be contained in the growth medium of the microalga (in particular by bacteria contained in this growth medium in embodiments of the invention wherein the growth medium is a raw effluent from a bioprocess, such as a raw effluent from a dark fermentation process). In other words, the method of the invention does not comprise any step of adding an enzyme or any other chemical substance capable of catalyzing a transesterification and/or esterification reaction in the growth medium of the microalga or in the hydroalcoholic solution, this not excluding configurations wherein such an enzyme would already be present in the growth medium in embodiments wherein this growth medium is an effluent from a bioprocess and is used as such, without any purification method (as will be described hereinbelow) for the cultivating step of the method of the invention.

By suitable growth medium, it is herein meant a growth medium containing all substances and nutrients required for microalgal growth. These substances and nutrients depend on the particular microalgal strain used in the method, as well as its suitable culture mode (mixotrophy, heterotrophy, etc.). It is within the ability of the person skilled in the art to select a suitable growth medium for a specific microalga strain implemented in the method of the invention, on the basis of his general knowledge.

Generally speaking, suitable growth media for microalgae comprise a nitrogen source, such as an ammonium salt, a phosphorus source and essential metabolic mineral salts, such as salts of potassium, sodium, calcium, magnesium, iron, etc. They may also comprise a carbon source and/or secondary nutrients such as vitamins and amino acids.

The pH of the growth medium is preferably between 6 and 9, more preferably between 6 and 7, for example of about 7.

In the context of the invention, the growth medium may be sterile. In particularly advantageous embodiments of the invention, the growth medium is not sterile. It may in particular contain bacteria.

It is also within the ability of the person skilled in the art to determine the conditions of the cultivating / cultivation step which are suitable to promote cell growth, according to the specific microalga strain involved. These conditions are preferably chosen so as to also promote lipid accumulation in the microalgal cells.

The cultivation step of the method of the invention is preferably advantageously carried out in mixotrophic conditions, for example in a photobioreactor or in an open pond, and in the presence of an organic carbon source in the growth medium.

The cultivating step is for example carried out at 25°C, preferably under constant light, for example at 100 µmol.m².s⁻¹, and preferably under constant agitation, for example at 130 rpm (in particular at 25 mm orbit), in particular when Erlenmeyer flasks are used as cultivation vessels, of for example 0.5 or 1 L volume, containing 0.2 and 0.4 L of culture medium, respectively.

In preferred embodiments of the invention, the cultivating step is carried out until a high amount of biomass has accumulated in the growth medium, and more preferably also until a high amount of lipids has accumulated in this biomass. In batch operating conditions, it can advantageously be carried out either until the complete consumption of the nutrients contained in the growth medium, or until the microalga reaches a stationary phase, i.e. after the end of the growth phase thereof. Preferably, the harvesting step is then performed when the cells have reached an early stationary phase.

The method of the invention can advantageously be carried out at low cost. It does not use any expensive exogenous enzymes or other chemical substances capable of catalyzing transesterification and/or esterification reactions. Furthermore, it is devoid of any drying step of the cell pellet obtained from the growth medium at the end of the cultivating step. According to the invention, this cell pellet is introduced in a wet state in the hydroalcoholic solution, which simplifies the implementation of the method by reducing the number of steps thereof. The C₁-C₄ alcohol can furthermore be used as such, in the sense that it does not need to be in an anhydrous form.

In preferred embodiments of the invention, the wet cell pellet is suspended in the hydroalcoholic solution immediately after its obtention, i.e., less than a few minutes after its obtention. Therefore, the suspending step and the incubating step are carried out within a few minutes after the concentrating step. This does not exclude embodiments wherein the wet cell pellet would be frozen immediately after its obtention, and kept at a temperature of -18°C or less, then introduced in the hydroalcoholic solution and thawed therein.

A C₁-C₄ alcohol is an alcohol molecule which comprises one to four carbon atoms. At least one, preferably each, C₁-C₄ alcohol implemented in the method of the invention is preferably a monohydric alcohol, i.e. having a single hydroxyl group. In preferred embodiments of the invention, at least one, preferably each, C₁-C₄ alcohol comprises one or two carbon atoms, i.e., is a C₁-C₂ alcohol. It is preferably methanol or ethanol. It can otherwise be propan-1-ol, propan-2-ol, butan-1-ol, butan-2-ol, 2-methylpropan-1-ol, 2-methylpropan-2-ol, etc. A mixture of several C₁-C₄ alcohols, in particular a mixture of methanol and ethanol, can also be used according to the invention.

The hydroalcoholic solution may or may not comprise alcohol(s) other than C₁-C₄ alcohol(s). In particular embodiments of the invention, it does not comprise any alcohol other than C₁-C₄ monohydric alcohol(s). Preferably, the hydroalcoholic solution does not comprise any organic solvent other alcohol(s). In particular, it preferably does not comprise chloroform.

The optimal pH of the hydroalcoholic solution depends on the particular microalgal strain used. This pH is generally preferably between 4.5 and 8.5, more preferably of about 7.

The incubating step of the method of the invention is carried out at a temperature between 10 and 60 °C. It can in particular be carried out at a temperature between 20 and 50 °C, more particularly between 20 and 45 °C, even more particularly between 30 and 45 °C, and most preferably between 30 and 40 °C. It can for example be carried out at about 30 °C. Here again, the person skilled in the art is capable of selecting the optimal temperature for the incubating step depending on the particular microalgal strain used in the method.

In the incubating step of the method of the invention, the addition of the C₁-C₄ alcohol, in the aqueous medium, to freshly harvested wet biomass triggers the transformation of lipids, in particular glycerolipids, into fatty esters and free fatty acids, but also the transformation of free fatty acids into fatty esters, solely by the action of the endogenous enzymes present in the microalga biomass. The fatty acyl chains are thereby directly functionalized in the biomass and they can then be separated from the reaction medium, the extraction of fatty esters and free fatty acids being in particular advantageously easier to realize than that of membrane glycerolipids.

The length(s) and unsaturation degree(s) of the fatty chain(s) of the fatty ester(s) and free fatty acid(s) obtained according to the invention depend on the particular microalga species implemented by the method. As an example, C18:2, C18:3 and C18:1 fatty esters can be obtained as reaction products. The method according to the invention may further respond to one or more of the features described below, implemented individually or in each of their technically operating combinations.

In particular advantageous embodiments of the invention, the growth medium for the cultivating step comprises a carbonaceous substance. It preferably comprises at least one of the following compounds: a C₁-C₆ fatty acid, lactate, ethanol, glucose, glycerol, carbon dioxide.

The growth medium preferably contains at least one, and possibly several, C₁-C₆ fatty acid(s).

Conventionally, C₁-C₆ fatty acids, also called short-chain fatty acids, or volatile fatty acids, are monocarboxylic acids comprising an aliphatic chain having 1 to 6 carbon atoms. As used herein, the term "acid" encompasses both the acid form and the carboxylate form of the compound.

In particular embodiments of the invention, the growth medium comprises, as C₁-C₆ fatty acid(s), at least acetate and/or butyrate and/or isobutyrate. It can also or otherwise comprise formate, propionate and/or pentanoate.

In particular embodiments of the invention, the method can comprise, after the cultivating step and before the harvesting step, a so-called post-culture step comprising incubating the cells in a suitable medium and under suitable conditions to promote the accumulation of lipids therein. Such a suitable medium may for example be a medium comprising an excess of carbon relatively to nitrogen and phosphorus, or even devoid of nitrogen and/or phosphorus (and comprising carbon). This suitable medium may be the growth medium used in the cultivating step of the method of the invention, after nitrogen and/or phosphorus exhaustion and, if necessary, addition of carbon; or it may be a new medium comprising carbon and devoid of nitrogen and/or phosphorus, wherein the cells recovered from the growth medium would be resuspended.

The steps of harvesting the cultivated cells (after the cultivating step or, if applicable, after the post-culture step) and concentrating them to obtain a wet cell pellet can be performed by any conventional means known to the person skilled in the art. In particular, the wet cell pellet can be obtained by centrifugation of the growth medium, and separation of the pellet from the supernatant.

The hydroalcoholic solution preferably comprises 15 to 50 % v/v, and more preferably 15 to 30% v/v of C₁-C₄ alcohol(s), in particular C₁-C₂ alcohol(s). Such a range of alcohol concentration ensures a high yield of fatty acid(s) esterification.

The biomass concentration in the hydroalcoholic solution is for example comprised between 25 and 80 g.L⁻¹.

The duration of the incubating step is comprised between 30 minutes and 48 hours, and preferably between 3 and 24 hours.

The step of recovering the fatty ester(s) from the cell suspension can be carried out by any conventional means known to the person skilled in the art. In particular embodiments of the invention, it is carried out by extraction of said ester(s) from said cell suspension, which is in an aqueous medium, with a non-polar aprotic organic solvent, such as hexane.

Any species of microalga can be used in the method of the invention. In particular embodiments of the invention, the microalga strain is a green microalga strain, more particularly a microalga strain belonging to the genus *Chlorella,* preferably to the species *Chlorella vulgaris* or *Chlorella sorokiniana.* Examples of such strains are the *Chlorella sorokiniana* strain available at the SAG culture collection under accession number SAG 211-8k and the *Chlorella vulgaris* strain available at the NIES culture collection under accession number NIES 227. In alternative particular embodiments of the invention, the microalga strain is phagotrophic. It can then belong to the genus *Ochromonas*, and preferably to the species *Ochromonas danica.* An example of such a strain is the *Ochromonas danica* strain available at the SAG culture collection under accession number SAG 933-7. Strains belonging to the genus *Euglena*, in particular the species *Euglena gracilis,* to the genus *Desmodesmus*, in particular the species *Desmodesmus armatus*, or the genus *Phaeodactylum*, in particular the species *Phaeodactylum tricomutum*, can also be used in the method of the invention, such a list not being exhaustive. Examples of such strains are the strains *Euglena gracilis* var. *bacillaris* available at the SAG culture collection under accession SAG 1224-5/15, *Euglena gracilis* var. *saccharophila* available at the Culture Collection of Algae and Protozoa (CCAP) under accession number CCAP 1224/7A, *Desmodesmus armatus* available at the CCAP collection under accession number CCAP 276/4D, and *Phaeodactylum tricornutum* available at the CCAP collection under accession number CCAP1052/1A.

The method of the invention can use a single microalga strain, or a mixture of several different microalga strains.

In preferred embodiments of the invention, the growth medium implemented in the cultivating step of the method contains bacteria, which can be of any type, and in particular a dark fermentation bacterial community. It has been discovered by the inventors that the presence of bacteria in the microalga growth medium can be beneficial for some microalgal strains, in particular with respect to biomass production and/or lipids accumulation within the microalgal strains, this being associated with a high rate of consumption of organic carbon contained in the growth medium. Without being bound by such a theory, it can be presumed here that there occurs in the growth medium a combination of several phenomena leading to such an advantageous result: the respiration of organic carbon and production of CO₂ by the bacteria, this carbon dioxide serving as a substrate for the growth of the microalga, the latter then producing dioxygen favoring the growth of the bacteria, and the use of bacterial biomass as a substrate by some microalga, this resulting in the advantageous valorization of all the organic carbon contained in the growth medium, especially when the method of the invention uses several microalgal strains simultaneously.

In particular embodiments of the invention, the growth medium is a synthetic medium.

In alternative, particularly advantageous, embodiments of the invention, the growth medium is an effluent from a bioprocess, and more particularly a dark fermentation effluent, naturally containing C₁-C₆ fatty acid(s), in particular acetate and butyrate, and often also containing lactate and ethanol. This effluent can be obtained from the dark fermentation of any organic carbonaceous feedstock. It is preferably obtained by the dark fermentation of food waste.

This dark fermentation effluent is preferably used directly, as such, in the sense that it is not subjected to any purification step, such as separation and/or filtration steps, nor to any sterilization step, in particular aiming at eliminating the bacterial community therefrom, before being used for the cultivating step of the method of the invention. Such a direct use of the effluent for the cultivating step of the method of the invention advantageously reduces the cost of implementation of the method, and, surprisingly, has no deleterious consequence on the performance thereof. On the contrary, as explained above, the presence of bacteria in the growth medium of the microalga can improve the accumulation of the latter.

Before being used, the dark fermentation effluent can be diluted with water, for example subjected to a 2-fold dilution, or it can be used undiluted, depending on the organic carbon and bacterial content thereof.

According to the particular composition of the effluent, and to the particular microalga strain used in the method of the invention, the effluent can, if needed, be supplemented with any of a pH adjuster, preferably sodium hydroxide NaOH or potassium hydroxide KOH, so as to obtain a pH of between 6 and 9, preferably between 6 and 7, a nitrogen source, such as ammonia, a phosphorus source, etc. It can for example be supplemented with NH₄ and KH₂PO₄, so as to obtain a C:N:P molar ratio which is optimal for growth, for example equal to 106:6:1.

Therefore, in particularly preferred embodiments of the invention, the method comprises prior steps of:
- performing dark fermentation of a feedstock, in particular an organic carbonaceous feedstock, preferably food waste, in anaerobic conditions using a suitable bacterial community,
- collecting an effluent from this dark fermentation, this effluent naturally containing C₁-C₆ fatty acid(s), and fermentation bacteria,
- and inoculating this effluent with cells of one or several microalga strain(s), the effluent then forming the growth medium for the cultivating step of the method of the invention. This inoculating step is preferably carried out without having subjected the effluent to any prior separation or sterilization step. It can optionally be carried out after having adjusted the pH of the effluent to a value of between 6 and 9, preferably between 6 and 7, and optionally after having supplemented the effluent with adequate substances.

The dark fermentation of the feedstock, in particular the organic carbonaceous feedstock, preferably of food waste, can be operated in any way known in the art. It can be operated in continuous mode. It can otherwise be operated in batch mode.

Dark fermentation processes are very well-known by the person skilled in the art. They are part of anaerobic digestion processes, wherein different bacteria perform hydrolysis of particulate waste matter, followed by dark fermentation and methanogenesis. Dark fermentation produces bio-dihydrogen, carbon dioxide and volatile fatty acids, typically acetate and butyrate. The invention advantageously takes advantage of these volatile fatty acids and this carbon dioxide, which are used by the microalga in the cultivating step of the method, thereby providing a new route of valorization of waste materials. As explained above, microalgae can grow fast on waste effluent products and accumulate high amounts of lipids which are valorized in the method of the invention *via* esterification and/or transesterification, for potential use in green chemistry or as biofuels.

Therefore, in its particular embodiments using dark fermentation effluents for microalgal growth, the method of the invention achieves production of lipid derivatives, more particularly methyl and/or ethyl fatty esters, from microalgae growing on dark fermentation effluents, in two innovative stages. First of all, the microalga is cultivated, preferably in mixotrophy, on the effluent of the dark fermentation of food waste. This effluent contains volatile fatty acids, such as butyrate, acetate, etc., and is preferably used raw, implying the presence of fermentative bacteria therein. After cell growth, immediately after harvesting and concentrating the cells contained in the growth medium, the *in situ* conversion of lipids into C₁-C₄ alkyl fatty ester(s), in particular methyl and/or ethyl fatty esters, is catalyzed by endogenous enzymes present in the microalgal biomass, after simple addition of the C₁-C₄ alcohol(s), such as methanol and/or ethanol, in the aqueous medium, and incubation of this reaction medium under stirring at the suitable temperature. This integrated method comprises very few steps and requires few intrants, so it can be implemented at low cost, and has a high biomass and lipids productivity, partly thanks to the consumption of 100% of the organic carbon of the initial feedstock. Furthermore, it is associated with low carbon dioxide emission, as carbon dioxide is metabolized by the microalga during the cultivating step.

The transesterification and/or esterification of microalgal lipids in the wet biomass produced from dark fermentation effluents, in the presence of the C₁-C₄ alcohol(s), such as methanol and/or ethanol, has been found to function with quite a variety of very phylogenetically different strains of microalgae, and found to work best with *O. danica* and *C. vulgaris.* On dark fermentation effluents from food wastes, the ratios fatty esters / fatty acids obtained at the end of the incubating step was found to be very high, the best ratio obtained being close to 80/20 with both these species.

The fatty esters produced by the method of the invention find many advantageous applications, in particular in the fields of green chemistry and of bio-fuels.

Another object of the invention is a device for implementing a method according to the invention, i.e., configured for implementing this method, for producing an ester of a fatty acid and a C₁-C₄ alcohol, in particular a C₁-C₂ alcohol, from an organic carbonaceous feedstock, in particular from food waste. This device comprises:
- a dark fermenter,
- a culture reactor configured for performing microalga culture under mixotrophy conditions, in particular comprising stirring means and lightning means, and optionally comprising aerating means,
- a system for collecting effluent from the dark fermenter and introducing it into the culture reactor,
- a system for harvesting microalga cells from a culture medium contained in the culture reactor and concentrating them into a wet cell pellet,
- a production reactor equipped with stirring means and heating means, and optionally aerating means,
- and a system for recovering fatty substances from an hydroalcoholic medium contained in the production reactor.

Each of these components of this device is conventional *per se*, and can be configured so as to be suitable for implementing methods responding to any of the features described above and their operating combinations.

The culture reactor can comprise two stages, namely a first stage configured for performing microalga culture under conditions promoting cell growth, and a second stage configured for performing microalga culture under conditions promoting lipid accumulation within the grown cells. The system for harvesting microalga cells from a culture medium contained in the culture reactor is then configured for harvesting the cells from the second stage of the reactor.

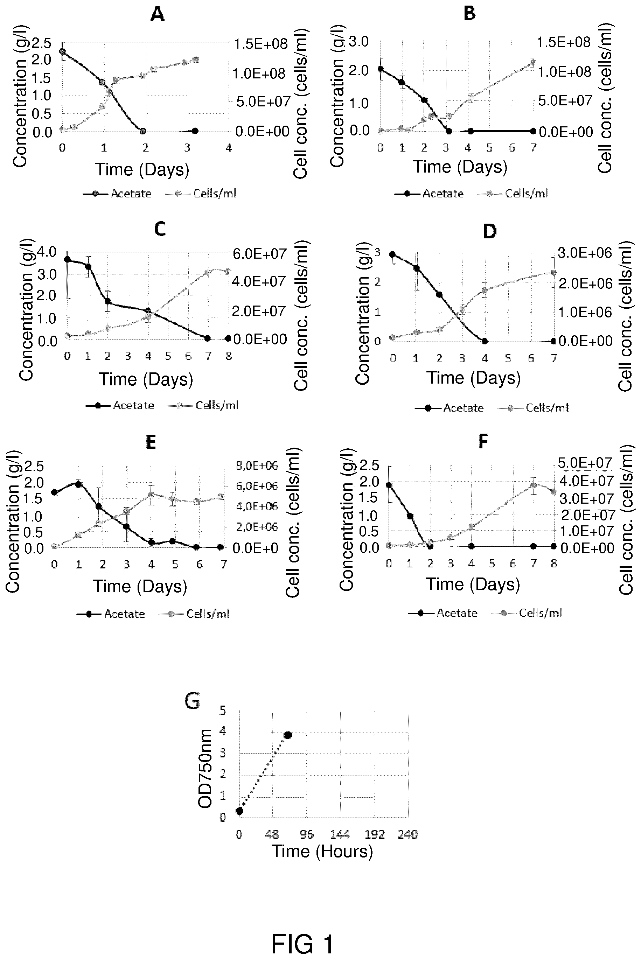

The features and advantages of the invention will emerge more clearly in the light of the following examples of implementation, provided for illustrative purposes only and in no way limitative of the invention, with the support of figures 1 to 16, in which:
- figure 1 shows the growth curves (cell concentration) and acetate consumption curves, of microalgae strains *C. sorokiniana* 211-8k (A), *C*. *vulgaris* (B), *D. armatus* (C), *E. gracilis* CCAP 1224/7A (D), *O. danica* (E) and *P. tricomutum* (F), and the growth curves (optical density at 750 nm OD750nm) of microalga strain *C. sorokiniana* 211-8k (G), these strains being cultivated on a synthetic growth medium comprising acetate,

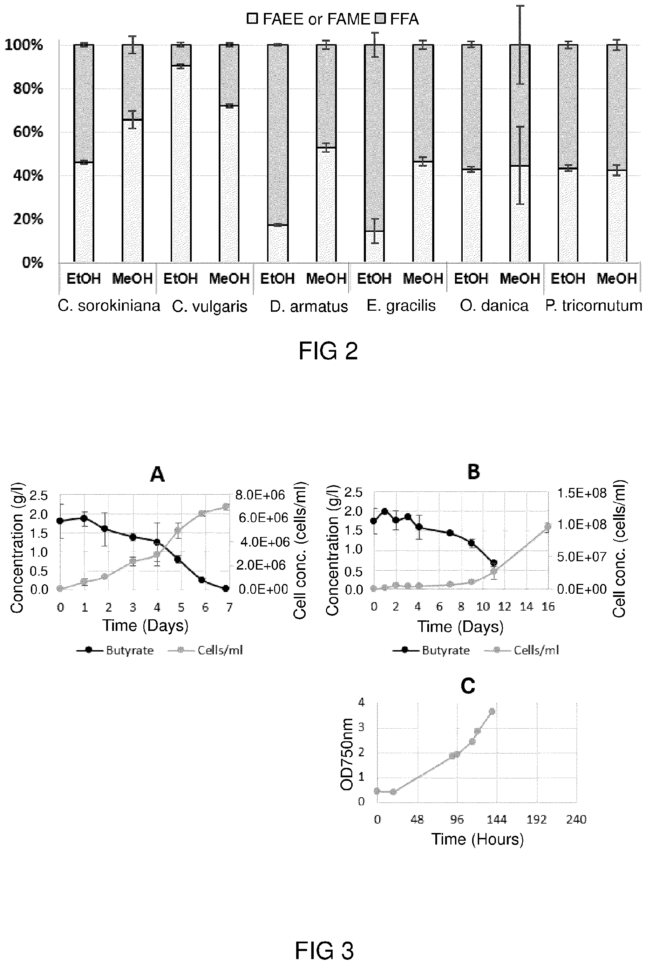

- figure 2 shows a graph bar representing the mass ratios of FFA and (FAEE or FAME) reported to the total fatty acid content ([FFA + FAEE] or [FFA + FAME]) recovered at the end of methods according to the invention using different microalgae strains, a synthetic growth medium comprising acetate and, as a C₁-C₂ alcohol, ethanol (EtOH) or methanol (MeOH) at 20 % v/v in the hydroalcoholic solution,
- figure 3 shows the growth curves (cell concentrations), and butyrate consumption curves, of microalgae strains *O. danica* (A) and *C. vulgaris* (B), and the growth curve (optical density at 750 nm OD750nm) of microalga strain *C. sorokiniana* 211-8k (C), these strains being cultivated on a synthetic growth medium comprising butyrate,

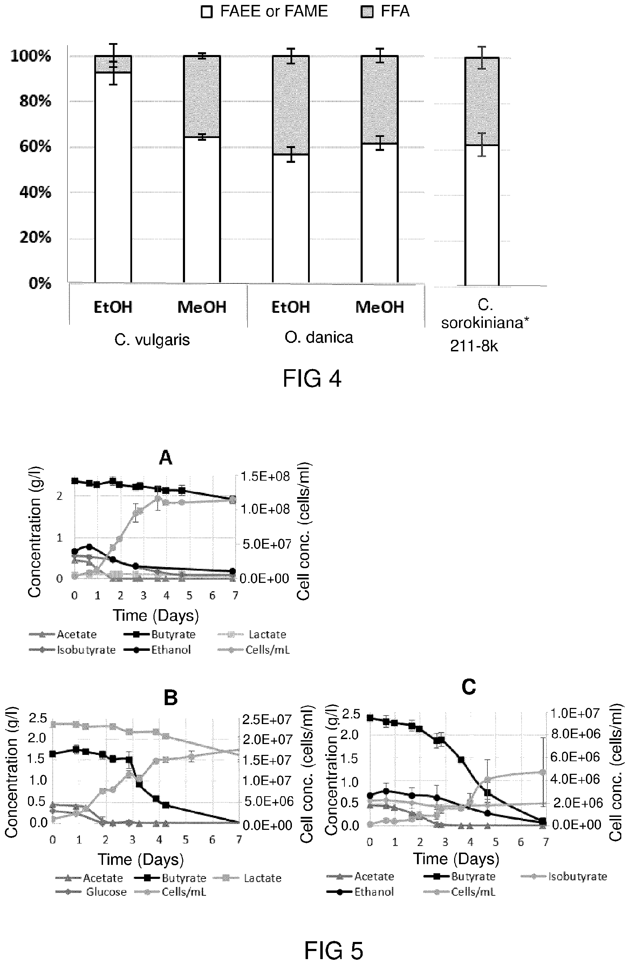

- figure 4 shows a graph bar representing the mass ratios of FFA and (FAEE or FAME) reported to the total fatty acid content ([FFA + FAEE] or [FFA + FAME]) recovered at the end of methods according to the invention using different microalgae strains, a synthetic growth medium comprising butyrate and, as a C₁-C₂ alcohol, ethanol (EtOH) or methanol (MeOH) at 20 % v/v in the hydroalcoholic solution, or (*) 15 % v/v ethanol + µbeads,
- figure 5 shows the growth curves (cell concentrations) and acetate, butyrate, isobutyrate, lactate and (residual) glucose consumption curves, of microalgae strains *C. sorokiniana* 211-8k (A), *O. danica* (B) and *E*. *gracilis* SAG 1224-5/15 (C), cultivated on a sterile glucose dark fermentation effluent,

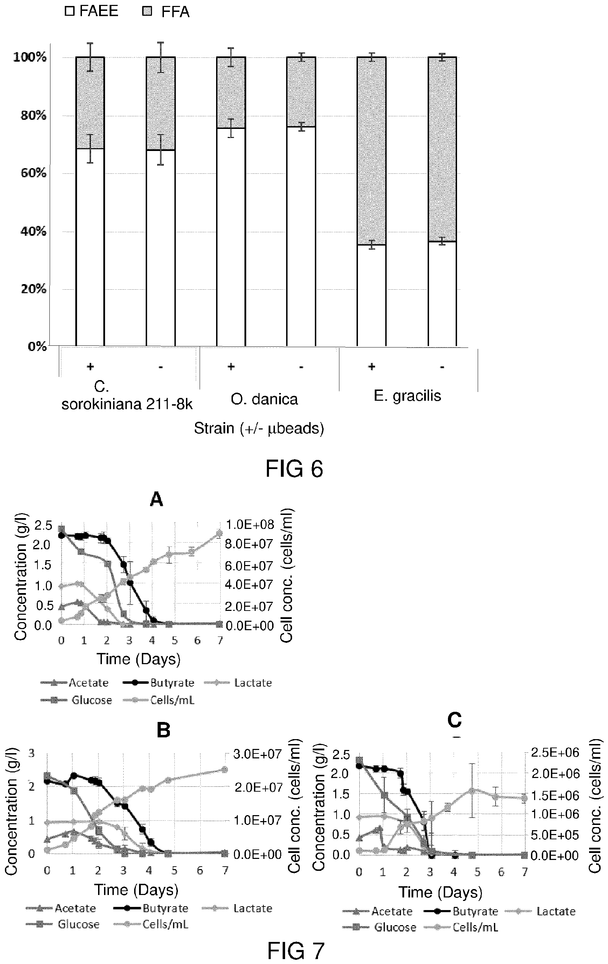

- figure 6 shows a graph bar representing the mass ratios of FFA and FAEE reported to the total fatty acid content [FFA + FAEE] recovered at the end of methods according to the invention using different microalgae strains, a sterile glucose dark fermentation effluent as growth medium and ethanol at 15 % v/v in the hydroalcoholic solution, with (+) or without (-) µbeads,
- figure 7 shows the growth curves (cell concentration), and acetate, butyrate, lactate and glucose consumption curves, of microalgae strains *C*. *sorokiniana* 211-8k (A), *O. danica* (B) and *E*. *gracilis* SAG 1224-5/15 (C), cultivated on a raw glucose dark fermentation effluent,

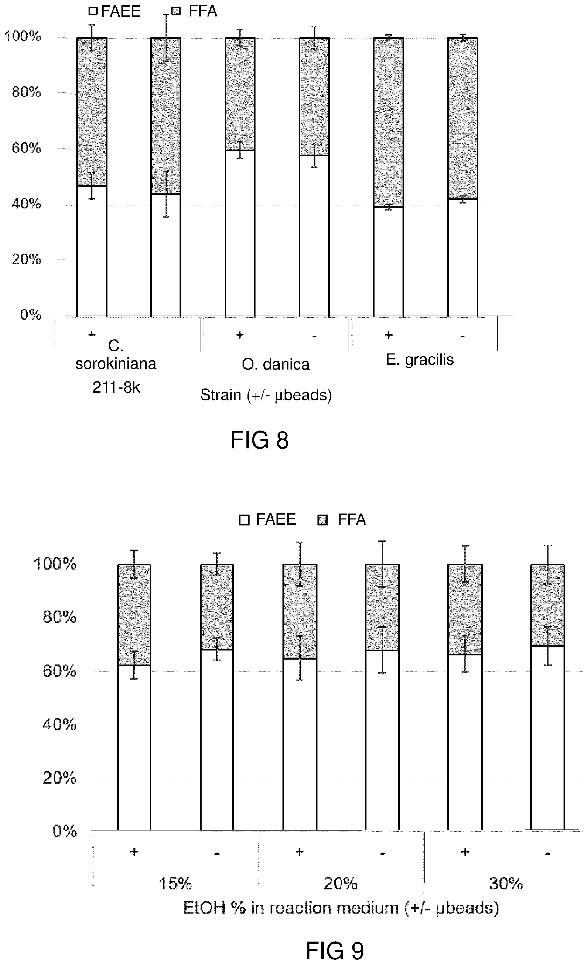

- figure 8 shows a graph bar representing the mass ratios of FFA and FAEE reported to the total fatty acid content [FFA + FAEE] recovered at the end of methods according to the invention using different microalgae strains, a raw glucose dark fermentation effluent as growth medium and ethanol at 15 % v/v in the hydroalcoholic solution, with (+) or without (-) µbeads,
- figure 9 shows a graph bar representing the mass ratios of FFA and FAEE reported to the total fatty acid content [FFA + FAEE] recovered at the end of methods according to the invention using an *O. danica* strain, a raw glucose dark fermentation effluent as growth medium and ethanol at 15%, 20 % or 30 % v/v in the hydroalcoholic solution, with (+) or without (-) µbeads,

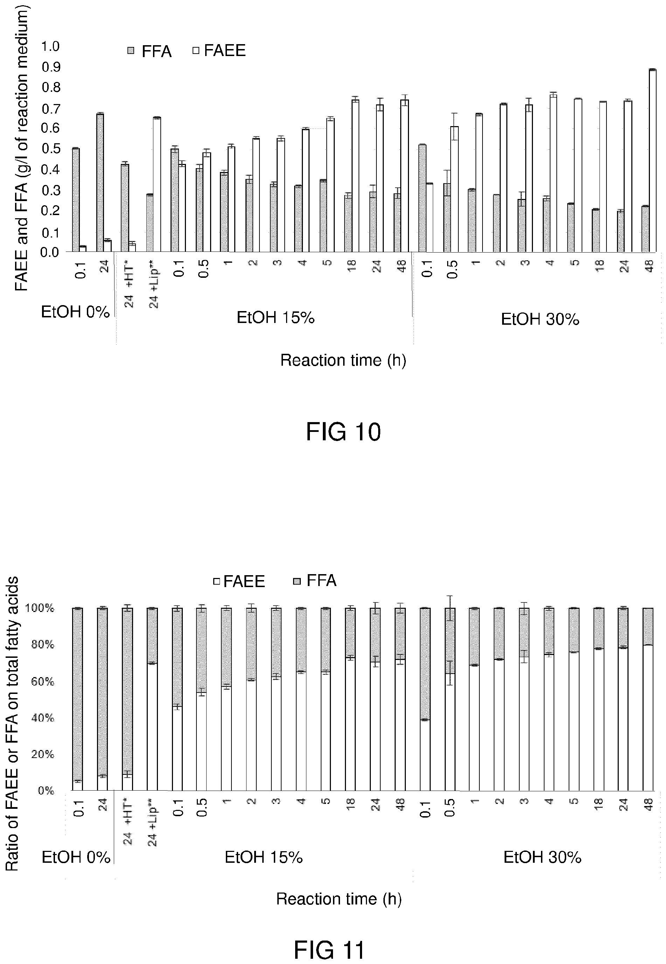

- figure 10 shows a graph bar representing the concentrations of free fatty acids (FFA) and fatty acid ethyl esters (FAEE) in the transesterification and/or esterification reaction medium at the end of methods according to the invention using an *O. danica* strain, a raw glucose dark fermentation effluent as growth medium and ethanol at 0 % (negative control), 15% or 30% v/v in the hydroalcoholic solution (HT*: Heat Treatment before reaction (100°C, 10 min), Lip**: addition of exogenous lipase/acyltransferase in the reaction medium),
- figure 11 shows a graph bar representing the mass ratios of FFA and FAEE reported to the total fatty acid content [FFA + FAEE] recovered at the end of methods according to the invention using an *O. danica* strain, a raw glucose dark fermentation effluent as growth medium and ethanol at 0 % (negative control), 15 % or 30 % v/v in the hydroalcoholic solution (HT*: Heat Treatment before reaction (100°C, 10 min), Lip**: addition of exogenous lipase/acyltransferase in the reaction medium),

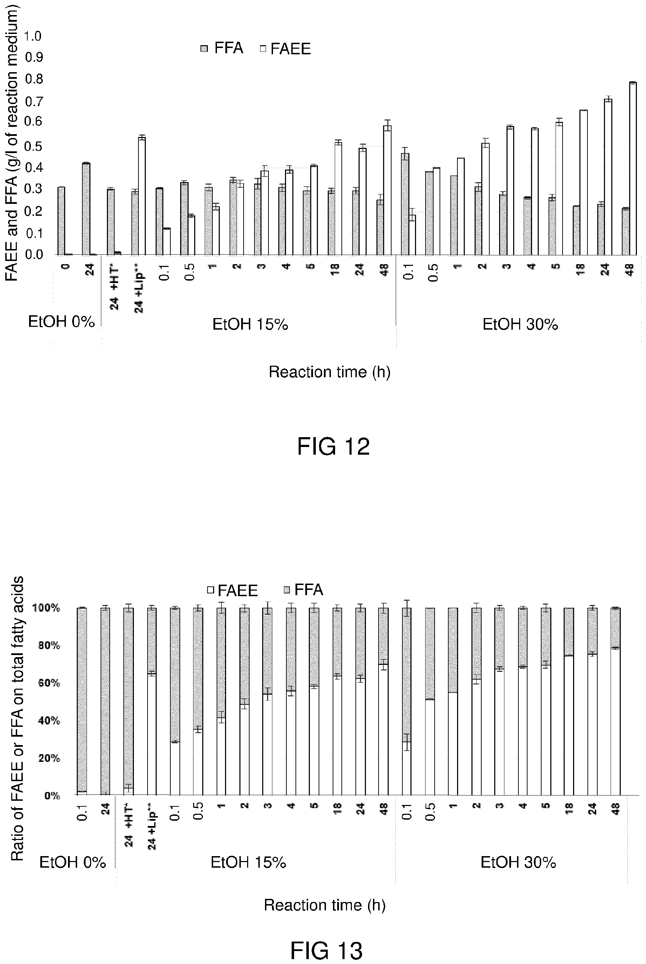

- figure 12 shows a graph bar representing the concentrations of free fatty acids (FFA) and fatty acid ethyl esters (FAEE) in the transesterification and/or esterification reaction medium at the end of methods according to the invention using a *C*. *vulgaris* strain, a raw batch dark fermentation effluent from food waste as growth medium and ethanol at 0 % (negative control), 15 % or 30 % v/v in the hydroalcoholic solution (HT*: Heat Treatment before reaction (100°C, 10 min), Lip**: addition of exogeneous lipase/acyltransferase in the reaction medium),
- figure 13 shows a graph bar representing the mass ratios of FFA and FAEE reported to the total fatty acid content [FFA + FAEE] recovered at the end of methods according to the invention using a *C*. *vulgaris* strain, a raw batch dark fermentation effluent from food waste as growth medium and ethanol at 0 % (negative control), 15% or 30% v/v in the hydroalcoholic solution (HT*: Heat Treatment before reaction (100°C, 10 min), Lip**: addition of exogenous lipase/acyltransferase in the reaction medium),

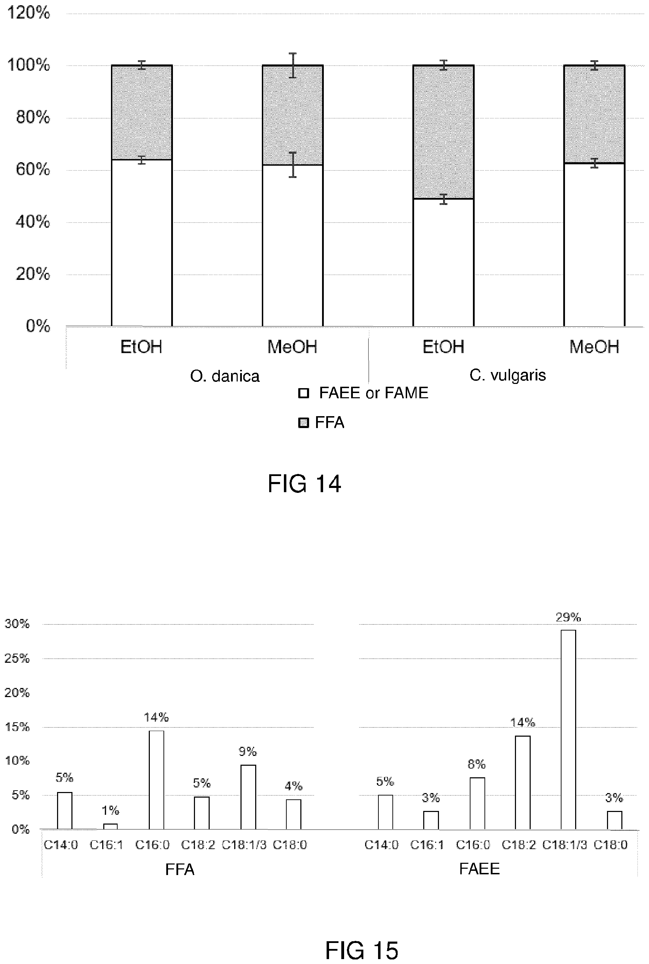

- figure 14 shows a graph bar representing the mass ratios of FFA and FAEE or FAME reported to the total fatty acid content ([FFA + FAEE] or [FFA + FAME]) recovered at the end of methods according to the invention using a *C*. *vulgaris* strain or an *O. danica* strain, an undiluted raw continuous dark fermentation effluent from food waste as growth medium and ethanol (EtOH) or methanol (MeOH) at 20 % v/v in the hydroalcoholic solution,
- figure 15 shows a graph bar representing the free fatty acids (FFA) and fatty ethyl esters (FAEE) profiles, expressed in percent of total fatty acid content ([FFA + FAEE]), recovered at the end of a method according to the invention using an *O. danica* strain, an undiluted raw continuous dark fermentation effluent from food waste as growth medium and ethanol at 20 % v/v in the hydroalcoholic solution

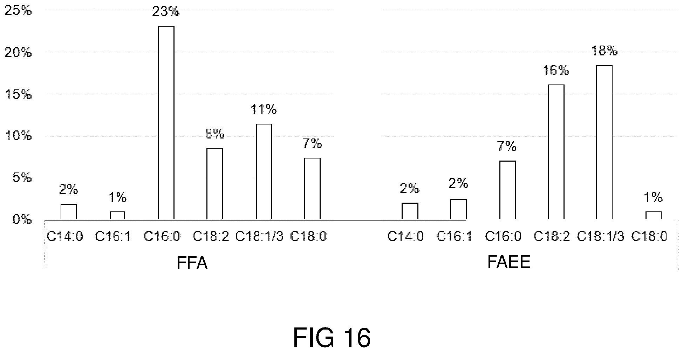

- and figure 16 shows a graph bar representing the free fatty acids (FFA) and fatty ethyl esters (FAEE) profiles, expressed in percent of total fatty acid content ([FFA + FAEE]), recovered at the end of a method according to the invention using a *C*. *vulgaris* strain, an undiluted raw continuous dark fermentation effluent from food waste as growth medium and ethanol at 20 % v/v in the hydroalcoholic solution.

### A/ Material and Methods

### A.1/ Microalgae strains

Seven strains were used in this study:
- *Chlorella sorokiniana* (Accession number: SAG 211-8k), *Euglena gracilis* var. *bacillaris* (Accession number: SAG 1224-5/15) and *Ochromonas danica* (Accession number: SAG 933-7), obtained from the SAG culture collection (Goettingen, Germany),
- *Chlorella vulgaris*, obtained from the NIES culture collection (Tsukuba, Japan) (Accession number: NIES 227),
- *Desmodesmus armatus* (Accession number: CCAP 276/4D), *Euglena gracilis* var. *saccharophila* (Accession number: CCAP 1224/7A) and *Phaeodactylum tricornutum* (Accession number: CCAP 1052/1A), obtained from the Culture Collection of Algae and Protozoa (CCAP, Scotland).

The strains were maintained on agar YEG medium for *O*. *danica,* on F/2A agar medium for *P. tricornutum* and on agar HAP medium for the others (compositions are given below), under constant illumination at 25 °C. Before performing experiments, few colonies were picked and re-suspended in 50 mL of the corresponding liquid medium in 125 mL flasks. Flasks were then incubated at 25°C, under constant light at 100 µmol.m².s⁻¹ and constant agitation of 130 rpm (2.5 cm orbit). Cells in exponential growth phase were used for further cultivations in dedicated medium.

### A.2 Growth media and fermentation protocols

### Synthetic media: HAP, HBP, YEG, F/2A

The HAP medium was composed as follows: 41.7 mM sodium acetate (1 gC.L⁻¹), 1 mM K₂HPO₄, 20 mM HEPES, 25 mL.L⁻¹ Beijerinck's (40x) solution, 1 mL.L⁻¹ Hutner's trace element solution and 100 µL.L⁻¹ vitamins solution. Beijerinck's solution consists of 300 mM NH₄Cl, 25 mM MgSO₄.7H₂O and 13 mM of CaCl₂; Hutner's trace elements solution is composed of 40 mM Na₂-EDTA.2H₂O, 20 mM FeCl₃, 10 mM MnCl₂.4H₂O, 4 mM ZnSO₄.7H₂O, 1 mM H₃BO₃, 1 mM CuSO₄.5H₂O, 1 mM NaMoO₄.2H₂O and 1 mM CoCl₂.6H₂O; the vitamins solution consists of 50 mM thiamine, 1 mM biotin and 1 mM cyanocobalamin. The pH of the medium was adjusted to 7.0 by addition of NaOH prior to sterilization by autoclave at 121 °C for 20 min. Vitamins were sterilized by filtration over a 0.2 µm filter and added after autoclaving to avoid their degradation. To obtain a solidified medium, 1.5% (m/v) agar was added before sterilization.

An HBP medium was also prepared by replacing sodium acetate of HAP by 20.8 mM sodium butyrate (1 gC·L⁻¹).

YEG medium is HAP medium without acetate but instead with 2.5 g of yeast extract and 5 g of glucose added, and no vitamins were used.

F/2A medium is composed of sea water with added 2 mM NaHCO₃, 5 mM HEPES buffer, 1 mM NaNO₃, 36 µM K₂HPO₄, 1 mL.L⁻¹ Hutner's trace elements. The pH was adjusted to 7.8 with NaOH and 0.1 mL.L⁻¹ of vitamins solution was added after sterilization.

### Model effluent from the dark fermentation of glucose

The composition of the fermentation medium is as follows: 100 mM of MES, 55.5 mM of glucose (10 g.L⁻¹), 5 mL.L⁻¹ of trace element solution, 2.5 mM NH₄Cl and 3 mM K₂HPO₄. The pH is adjusted to 6 using NaOH. The trace element solution contains 10 mM FeCl₂.H₂O, 0.75 mM H₃BO₃.H₂O, 7 mM MnSO₄.H₂O, 0.1 mM CoCl₂.6 H₂O, 5 mM ZnCl₂, 1 mM NiCl₂.6H₂O, 9 mM CuCl₂.2H₂O, 1.2 mM NaMoO₄.2H₂O. The inoculum used for these fermentations was obtained from activated sludge (AS) of the waste water treatment plant at Narbonne, France. This AS is centrifuged then freeze-dried to obtain an inoculum in powder form which is then stored at -80°C. The inoculum used contains 75% volatile matter (VM). Before use, an aliquot of inoculum is thawed at room temperature then suspended in Milli-Q water to obtain a biomass concentration of 25 gVM/L. This solution is then pretreated by 15 min thermal shock at 90°C. This thermal shock makes it possible to select sporulating bacteria and H₂ producers.

The dark fermentation process was carried out under anaerobic conditions in a total volume of 250 mL in 600 mL bottles hermetically closed with a septum. A volume of 10 mL of the inoculum is introduced into each flask containing 240 mL of medium. A biomass concentration of 1 gVM/L and a glucose concentration of 10 g.L⁻¹ yields a substrate:biomass (S:X) ratio of 10 at the start of dark fermentation. An anaerobic atmosphere is obtained by flushing N₂ for 10 min through the headspace of the bottles. Cultures were monitored online by measuring the pressure and analyzing the gases produced throughout the culture using a micro gas chromatography system (described below). To allow this monitoring, the internal volume of the bottles is determined beforehand by measurement of the pressure difference before and after adding a known volume of air (PV=nRT). The volume of headspace is determined by subtracting, from the volume of the bottle, the volume of medium added. Fermentation cultures were done at 37°C and stopped when the production of H₂ and CO₂ stabilized.

### Real effluent from the dark fermentation of food waste

The feedstock for the dark fermentation consisted of a reconstituted food waste soup prepared directly from frozen foods, i.e. minced beef (13 g.L⁻¹), yogurt (9 g.L⁻¹), red berries (13 g.L⁻¹), breaded fish (9 g.L⁻¹), French fries(17 g.L⁻¹), mix of vegetables (13 g.L⁻¹) and bread (13 g.L⁻¹). The composition was inspired by an average biowaste composition of major food retailers in France. The total COD of the feedstock was measured using the Vario MR COD Reagent. Subsequently, thermal hygienization was done for 1 h at 70 °C in a water bath. Both the dilution and hygienization processes were carried out to fulfil European law requirements for biowaste, and to mimic industrial procedures to eliminate pathogens that might exist in food waste. The resulting mixture was sieved through a 2 mm mesh sieve and diluted again with tap water, reaching 27 ± 3 gTS.L⁻¹ (total solids) 26 ± 4 gVS.L⁻¹ (volatile solids) and 33 ± 4 g.L⁻¹ COD prior to fermentation. The feedstock solution was stored at 4 °C and prepared every four days. For batch operation only, 100 mM MES buffer was added and the pH adjusted to 6.

The experimental dark fermentation setup is as follows. Experiments were set up in 3 L glass fermenters (Applikon Bio). Temperature and pH were monitored and controlled online by an M300 system (Mettler Toledo). The temperature was measured with an immersed probe and regulated by a heating blanket. The pH was measured by an *in situ* probe and was regulated with 3 M NaOH. As indicated above, for batch operation the pH was controlled via the addition of MES buffer. Mixing was ensured by mechanical stirring. Pressure was regulated with a control device combining a pressure sensor (LEO3, Keller) and a peristaltic pump (Masterflex L/S 7554-85, Cole Parmer) following a two-band control law between 1030 and 1070 mbar. Gas production was measured through the pressure variations inside the fermenter and by using the operating time of the calibrated peristaltic pump. Produced gas volumes were normalized (T = 0 °C and p = 1 atm).

To run the reactors in batch mode, inoculum (as described above), feedstock (substrate), and tap water were added to reach the working volume of 2.2 L and a headspace of 0.8 L. Operation was started with a S:X ratio of 7.8 (VS.VS⁻¹) and a substrate concentration of 7.8 gVS.L⁻¹ (10 gCOD.L⁻¹). Fermenters were flushed with nitrogen for 15 min to remove O₂ and ensure anaerobic conditions. The temperature was monitored and regulated at 37 °C, and stirring was set at 180 rpm. The effluent is collected when H₂ production reaches a plateau, as monitored via microGC (see below), which corresponds to 3-4 days.

For continuous operation of the dark fermentation, the inoculum, feedstock (substrate), and tap water were added to reach the working volume of 2 L with headspace of 1 L. The reactors were first operated in batch mode for 9 h. Initial batch operation was started with a substrate to biomass ratio of 9 (VS.VS⁻¹) and a substrate concentration of 7.8 gVS.L⁻¹ (10 gCOD.L⁻¹). Fermenters were flushed with nitrogen for 15 min to remove oxygen and ensure anaerobic conditions, which were confirmed by oxidation reduction potential (ORP) values lower than -400 mV during the entire continuous process. pH and temperature were regulated and monitored at 5.51 ± 0.02 and 36.9 ± 0.3 °C, respectively, and stirring was set at 180 rpm. After this batch mode phase, continuous operation was initiated by starting a programmable peristaltic pump (Masterflex L/S model 07522-30, Cole Parmer) and head pump (Masterflex L/S ^{®} FV-07018-20, Cole Parmer) configurated to deliver 100 mL of feed solution (26 gVS·L⁻¹) every 36 min. The organic loading rate (OLR) and hydraulic retention time (HRT) during continuous operation were 60 gVS·L·d⁻¹ (77 gCOO·L·d⁻¹) and 12 h, respectively. Effluents from the dark fermentation stage were collected, without any separation or filtration, and stored at -20°C until further use, or were used directly for further cultivation of microalgae.

### Gas and metabolite analysis

The outlets of the fermentation reactor vessels were connected to a micro gas chromatograph (GC R3000, SRA Instruments) for online gas composition monitoring. The chromatograph was equipped with a column for CO₂ analysis (Molesieve 5A 10 m column, temperature of 80 °C, 30 psi with argon as carrier gas) and a second column for N₂, CH₄, H₂, and O₂ analysis (PoraPlot U 8 m column, temperature at 70 °C, 20 psi with helium as carrier gas). Two micro-thermal conductivity detectors set at 90 °C were used. Supernatants of centrifuged liquid samples were filtered through 0.2 µm nylon filters before metabolite analysis. Volatile fatty acid (VFA) concentrations of each sample were measured using a Clarus 580 gas chromatograph (Perkin Elmer) equipped with an Alltech FFAP EC^{™} 1000 column coupled to a flame ionization detector (FID) at 280 °C. The carrier gas was N₂ circulating at a flow rate of 6 mL.min⁻¹. High Performance Liquid Chromatography (HPLC) was used to analyze other metabolites (ethanol, non-VFA organic acids, residual sugars). A protective precolumn (Micro-Guard cation H Refill Cartridges, Bio-Rad) was used and then an HPX-87H column (300 × 7.8 mm, Bio-Rad) running at 35 °C coupled to a refractive index detector (R410, Waters) at 45 °C. A solution of 4 mM H₂SO₄ at 0.3 mL.min⁻¹ was used as eluent.

### A.3/ Microalgae batch cultivation

### Effluent preparation for microalgae cultures

To enable the growth of microalgae, the effluent was supplemented with NH₄ and with KH₂PO₄ so as to respect the molar ratio C:N:P = 106:6:1, except for the undiluted raw effluents implemented in Example 3, where no additions were made. Furthermore, 1 mL.L⁻¹ of Hutner's solution was also added and the pH adjusted to 7. To cultivate the microalgae in the presence of dark fermentation bacterial communities, the effluent was used directly. To cultivate microalgae in the absence of bacteria, the medium was first centrifuged for 10 min at 8000xg. The supernatant was recovered and passed over a 0.22 µm filter. This sterile dark fermentation effluent (DFE) was then stored at 4°C for later use. Samples for gas chromatography (GC) and HPLC analysis were carried out on the DFE as described above.

### Culture conditions

For the cultivation of microalgae, the sterile synthetic medium or the raw or filter-sterilized DFE containing the additions indicated above were transferred to sterile Erlenmeyer flasks, either 200 mL DFE in 500 mL flasks or 400 mL DFE in 1L flasks. The optical density of the precultures was measured at 750 nm (OD750nm) in order to avoid any interference by microalgal pigments. The inoculation was carried out with a concentrated preculture resuspended in phosphate buffered saline (PBS) so as to obtain an initial OD750nm of 0.1 in each flask. The different cultures were then placed in conditions identical to those described for the precultures. The pH of real effluents was adjusted to 7 once a day with a 5M HCl solution (absence of buffer). The culture was stopped after complete consumption of the acetate and butyrate in the effluent or when the microalgae reach a stationary phase.

### Biomass analysis

Biomass production was determined by dry weight determination (DW) after filtration of 2-20 mL of samples (depending on biomass concentration) over a dried Whatman^{®} GF/C glass microfiber filter (1.2 µm) and overnight drying at 105 °C. Cell counting was also performed using a Malassez counting chamber and immobilizing motile cells with 0.01% Lugol (*E*. *gracilis*) or 0.01% formaldehyde (*O*. *danica*)*.* Total lipids were measured by the phosphovanillin method (Mishra et al., 2014, Bioresour. Technol. 155, 330-333). Phosphovanillin reagent was freshly prepared before analysis by dissolving 0.6 g vanillin in 10 mL ethanol, 90 ml deionized water and 400 mL of H₃PO₄ (85 %). The resulting reagent was stored in the dark until use. First, 2 mL of H₂SO₄ (98 %) were added in the tubes containing microalgae samples. The tubes were heated 10 min at 100°C. After cooling on ice, the reaction was initiated by addition of 5 mL of phosphovanillin reagent prior incubation for 15 min at 37°C. Tubes were periodically shaken by inversion and were then left in the dark at room temperature for 45 min for color development. After cooling, absorbance of suspensions was measured at 530 nm with an Aqualytic^{®} spectrometer and compared to distilled water. Calibration curves were obtained using canola oil.

Metabolite analysis in the liquid medium were measured by GC and HPLC as described above.

### A.4/ Transesterification and/or esterification of lipids in wet microalgae biomass

30 mL of microalgae cultures were centrifuged at 4000xg during 10 min. The supernatant was discarded and the cell pellet was resuspended in aqueous solutions with ethanol or methanol at concentrations ranging from 15 to 50% v/v, with biomass concentrations in the reaction medium from 25 to 80 g.L⁻¹. For experiments with microbeads addition ("+ µbeads") 200 mg of 0.1 mm glass beads were added to the reaction medium. The suspensions were incubated at 30°C under magnetic stirring for 0.5 to 48 h. Afterwards, to stabilize the microalgae biomass composition, the cell suspensions were heat treated at 100°C for 10 min before freezing at -20°C. To serve as reference for further lipid analysis, also a cell pellet deriving from 10 mL of culture was heat treated directly and frozen.

### A.5/ Quantification of fatty esters and free fatty acids

### Extraction of fatty acid esters and free fatty acids

500 µL of each reaction medium was transferred to a Wheaton^{®} vial with 50 µL of internal standard, consisting of a mix of ethyl pentadecanoate C₁₅ ethyl ester and heptadecanoate C₁₇ fatty acid at a concentration of 8.8 mM. 75 µL of MeOH/sulfuric acid (14:1) was added together with 0.1 mg/mL of butylhydroxytoluene (BHT) to 500µL of hexane. Glass beads were added before incubation at 40°C and 500 rpm overnight. After centrifugation at 16000g during 10 min at 4°C, the organic phase was recovered and kept in a new vial. The remaining aqueous phase was homogenized and 500 µL of hexane was added in order to do a second extraction during 2 h. Organic phases were pooled and 200 µL was mixed with 25 µL of MSTFA (N-Methyl-N-(trimethylsilyl)trifluoroacetamide) and 25 µL of pyridine for derivatization. The mix was heated at 50°C during 20 min before analysis by GC-FID.

### Analysis of fatty acid esters and free fatty acids by GC-FID

Gas chromatography coupled with a flame ionization detector (GC-FID) was used for lipid analysis to quantify and identify free fatty acids and fatty acid ethyl/methyl esters. The GC analysis was performed using a Shimadzu GC2010 Plus chromatograph equipped with an automatic sampling system (AOC 20i), a temperature programmable injector (PTV) with flow splitting, an FID and a Zebron^{™} ZB-5HT^{®} polyimide-coated fused silica phase capillary column (15 m long × 0.25 mm diameter × 0.1 µm thick) for high temperature analysis (Phenomenex). The signal was recorded and analyzed by LabSolutions software (Shimadzu). Analytical parameters were as follow: helium flow rate (mobile phase): 1 mL.min⁻¹, split ratio: 5; analyzed temperatures injector: gradient from 290 to 400°C (200°C.min⁻¹) and 36.45 min hold at 400°C; oven: gradient from 150 to 230°C (5°C.min⁻¹), then from 230 to 250°C (2°C.min⁻¹), then, from 250 to 390°C (35°C.min⁻¹) and 5 min hold at 400°C; FID detector: 400°C; sample injection volume: 0.5 µL. Calibration curves were realized using mixtures of ethyl/methyl esters and fatty acids (C₁₂, C₁₄, C₁₆, C16:1, C16:2, C₁₈, C18:1, C18:2, C18:3) within a concentration range from 0 to 4 mM, prepared according to the same protocol as for the enzymatic reactions but without the wet biomass.

### B/ Results

The 7 different strains, belonging to a variety of families and genera, were tested for their productivity when cultivated on synthetic medium and dark fermentation effluents (DFE) and/or for the ability of their endogenous enzymes to catalyze *in situ* transesterification and/or esterification reactions in the presence of low level of ethanol or methanol.

The first proofs of concept were carried out on synthetic media representative of dark fermentation effluents, then experiments were carried out on effluents of increasing complexity, and finally on real DFE.

### B.1/ Example 1 - Cultures on synthetic effluents and production of fatty acid esters (FAE) and free fatty acids (FFA)

### B.1.1/ Synthetic acetate medium

In order to test the 7 different strains for their capacity to transesterify and/or esterify their lipids, biomass was first produced on a standard growth medium containing acetate (HAP medium). Acetate is one of the main components of DFEs and is well adapted to mixotrophic growth of many microalgae.

### Growth curves

The cell concentration or optical density at 750 nm (OD750nm) and the acetate content in the HAP medium were monitored over time. The curves are shown in figure 1. All strains are able to grow on HAP medium and show mixotrophic capacity as deduced from their acetate uptake. It is to be noted that all cultures were axenic cultures, except for *P*. *tricomutum* which contained also bacteria.

### Fatty acid esters and free fatty acids production

In the following experiments, after cultivating the different strains on HAP medium, the culture was stopped after complete consumption of the acetate or when the microalgae reached a stationary phase. The cells were harvested and concentrated by centrifugation. Pellets were resuspended in hydroalcoholic solution to reach a 20- to 30-fold cell density (15 to 85 gDM/L) and a concentration of 20% v/v ethanol or methanol. Concentrated hydroalcoholic cell suspensions were incubated under magnetic stirring (400 rpm) at 30°C during 24h to assess *in situ* transesterification and/or esterification, e.g. the conversion of the biomass lipids into fatty acid methyl or ethyl esters (FAME and FAEE) and free fatty acids (FFA). After their extraction from the culture medium, these substances were quantified by GC-FID.

The mass ratios of FFA and (FAEE or FAME) reported to the total mass of fatty acid content ([FFA + FAEE] or [FFA + FAME]) are shown in figure 2. It appears that the strains displaying the highest potential for *in situ* transesterification and/or esterification are *C. vulgaris* and *C. sorokiniana,* with both ethanol and methanol as co-substrate for the transesterification and/or esterification reactions, allowing ester ratios from 45% to 90%. Transesterification and/or esterification, although at a lower ratio, were also obtained with *D. armatus* and *E. gracilis* and with *O. danica* and *P. tricornutum.* Depending on the strain, the use of ethanol or methanol resulted in significant differences or comparable results in the ratios.

Besides, total fatty acid content (sum of FAEE and FFA quantified by GC-FID after extraction) were quite low in biomasses of *D. armatus*, *E. gracilis* and P. *tricornutum* (< 1% and < 1g/L of reaction medium) and reached maximum of 4.5 % to 9 % of dry mass in *C. vulgaris* and *O. danica* respectively (1.4 g/L of reaction medium for both).

Altogether these results on HAP medium suggest that all tested strains achieve fatty acid esters production through *in situ* transesterification and/or esterification in the presence of ethanol or methanol, with *Chlorella* strains (especially *C*. *vulgaris*) and *O. danica* exhibiting the highest potential for such production.

### B.1.2/ Synthetic butyrate medium

### Growth curves

Several strains (*C*. *vulgaris*, *C. sorokiniana* and *O. danica*) were tested for their capacity to grow on HBP medium. The cell concentration or optical density at 750 nm (OD750nm) and the butyrate content in the HBP medium were monitored over time. The curves are shown in figure 3. All strains are able to grow on the HBP medium and perform butyrate uptake.

### Fatty acid esters and free fatty acids production

In the following experiments, after cultivating the different strains on HBP medium, the cells in stationary phase were harvested and concentrated by centrifugation. Pellets were resuspended in hydroalcoholic solution to 15 to 25 gDM/L and a concentration of 20% v/v ethanol or methanol, or, for the strain *C. sorokiniana*: 15 % v/v ethanol + µbeads. Concentrated hydroalcoholic cell suspensions were incubated under magnetic stirring (400 rpm) at 30°C during 24h to assess *in situ* transesterification and/or esterification, e.g. the conversion of the biomass lipids into fatty acid methyl or ethyl esters (FAME and FAEE) and free fatty acids (FFA). After their extraction from the growth medium, these substances were quantified by GC-FID.

The mass ratios of FFA and (FAEE or FAME) reported to the total fatty acid content ([FFA + FAEE] or [FFA + FAME]) are shown in figure 4. These results confirm that the strain displaying the highest potential for *in situ* transesterification and/or esterification is *C. vulgaris,* with 92.5% of ethyl esters formed in the presence of 20% of ethanol as co-substrate, also confirming higher transesterification and/or esterification efficiency with ethanol than methanol for this strain. With the biomass of *O*. *danica*, results obtained in the presence of ethanol and methanol are comparable (about 60% of esters). Interestingly, ester ratios are also higher for the strain *O. danica* compared to those obtained after cultivation on acetate (figure 2), suggesting that the presence of butyrate in the medium is particularly favorable to the formation of fatty acid esters by endogenous enzymes of this species. Furthermore, maximum total fatty acid contents in the reaction medium were comparable to those obtained from HAP cultivations, and of about 5 % to 7 % of the dry matter mass in the production medium.

### B.2/ Example 2 - Model dark fermentation effluents

In this experiment the cultivation of microalgae is carried out on real DFEs but using as feedstock glucose (and not food waste). This represents an intermediate approach where the effect of the presence of fermentative bacteria and their metabolites can be investigated in comparison to conditions where bacteria were removed.

### B.2.1/ In the absence of bacteria

Cultivation of microalgae on filter-sterilized glucose DFEs allows to verify growth in absence of bacteria but in presence of their fermentative metabolites, and is an indication of the chemical compatibility of the DFEs with microalgae growth.

### Growth curves

The model Glc-DFE (obtained from glucose) was used for microalgae cultivation, without dilution, and after filtration on 0.45 µm filters.

The strains *C. sorokiniana* 211-8k, *O. danica* and *E*. *gracilis* SAG 1224-5/15 were tested in this experiment.

The cell concentration of the cultures was monitored over time, as well as the fermentation metabolites (acetate, butyrate, lactate, glucose) consumption. The curves (figure 5) show that all the strains tested are able to grow on this medium, and reached higher density than on synthetic media HAP or HBP (owing to higher organic substrate concentration in the media) with final dry matter superior to 2.7 g/L. Metabolites were consumed differently and at different rates by the different strains. All species exhibit multiple substrate mixotrophy, with lactate being consumed poorly.

### Fatty acid esters and free fatty acids production

After cultivation of *O*. *danica, E. gracilis* and *C. sorokiniana* 211-8k on the Glc-DFE model effluent (without bacteria), the cells in stationary phase were harvested and concentrated by centrifugation. Pellets were resuspended in hydroalcoholic solution to reach a 20- to 30-fold cell density (60 to 80 gDM/L) and a concentration of 15% v/v ethanol. Concentrated hydroalcoholic cell suspensions were incubated under magnetic stirring (400 rpm) at 30°C during 24h to assess *in situ* transesterification and/or esterification. For the *in situ* transesterification and/or esterification reactions, two conditions were compared: with (+) or without (-) µbeads.

The results obtained from cultivation on this model dark fermentation effluent from glucose (Glc-DFE) without bacteria, followed by *in situ* transesterification and/or esterification with 15 % v/v ethanol, shown in figure 6, confirm the interesting ability of the tested strains, in particular the *C. sorokiniana* strain as well as that of *O. danica,* to produce fatty acid ethyl esters (FAEE) in these conditions: 68% and 76% of ethyl ester ratios were obtained from respectively *C. sorokiniana* 211-8k and *O. danica.* Cultivation of *O. danica* on the model Glc-DFE thus allows increasing ethyl ester ratio compared to those obtained on HAP and HBP. The use of microbeads did not induce significant change in the FAEE production.

### B.2.2/ In the presence of bacteria

Cultivation of microalgae on raw glucose DFEs allows to verify growth in the presence of bacteria, and is an indication of the overall compatibility of the DFEs with microalgae growth.

### Growth curves

The model Glc-DFE (obtained from glucose) was used directly for microalgae cultivation, without dilution and without filtration. Bacteria from dark fermentation were thus present in the effluent.

The strains *C. sorokiniana, O. danica* and *E*. *gracilis* SAG 1224-5/15 were tested in this experiment.

The cell concentration of the cultures was monitored over time, as well as the fermentation metabolites (acetate, butyrate, lactate, glucose) consumption. The curves (figure 7) show that all the strains tested are able to grow on this medium, and reached higher density than on synthetic media HAP and HBP with final dry matter superior to 2.5 g/L. Due to the presence of bacteria, metabolite consumption was increased and was complete. Although the microalgae have therefore less access to the organic substrate, bacterial respiration produces CO₂ which benefits microalgae directly. In this case, growth of *O*. *danica* was higher in presence of bacteria, which may result from its bacteriovory capacity.

### Fatty acid esters and free fatty acids production

After cultivation of *O. danica, E. gracilis* and *C. sorokiniana* on Glc-DFE with bacteria, the cells in stationary phase were harvested and concentrated by centrifugation. Pellets were resuspended in hydroalcoholic solution to reach a 20- to 30-fold cell density (50 to 73 gDM/L) and a concentration of 15% v/v ethanol. Concentrated hydroalcoholic cell suspensions were incubated under stirring at 30°C during 24h to assess *in situ* transesterification and/or esterification. Two conditions were compared: with (+) or without (-) µbeads. The results obtained from cultivation on this model dark fermentation effluent (Glc-DFE) in the presence of bacteria followed by transesterification and/or esterification, shown on figure 8, are comparable to those obtained from the medium without bacteria, the ester ratios being slightly smaller. The higher FAEE ratios were again obtained with *O. danica* (59%) followed by that from *C*. *sorokiniana* (43%). The FAEE ratio from *E*. *gracilis* biomass were higher than previously (41%). The final total fatty acids content in the reaction medium were satisfactory (0.43 to 1.04 g/L with µbeads for example).

### B.2.3/ Different ethanol concentrations with O. danica

To investigate the potential effect of ethanol concentration, the same experiment was implemented again with *O. danica,* (cultivation on raw Glc-DFE used directly, without dilution or filtration).

At the end of growth, the cell density was 1.1×10⁷ cells/mL. Acetate and butyrate were completely consumed within 8 days of culture.

Biomass harvesting was followed by *in situ* transesterification and/or esterification in the presence of 15%, 20% or 30% v/v ethanol with and without microbeads addition. In all conditions the medium was mixed by magnetic stirring, at 30°C during 24h.

The results, presented in figure 9, show that increasing ethanol concentration does not allow to increase the FAEE ratio, which remains in the range of 66% in all conditions. The total fatty acid content in the reaction medium is 0.4 g/L. The transesterification and/or esterification yield is not limited by ethanol concentration in the tested range of 15 to 30 % v/v.

### B.3/ Example 3 - Real dark fermentation effluents from food waste

The last step in the demonstration of the feasibility of the method of the invention comprising growing microalgae on DFEs is the use of effluents obtained from the dark fermentation of real waste, the method of the invention then closely resembling an actual industrial process. In the following experiments, the model dark fermentation effluents were obtained from food waste (FW-DFE). FW-DFE were not filtered for microalgae cultivation, so bacteria from DF were always present in the medium. The *O. danica* and *C*. *vulgaris* strains were tested for growth and transesterification and/or esterification on FW-DFE from either batch or continuous dark fermentation conditions.

### B.3.1/ Cultivation of the microalgae on effluents from batch DF

FW-DFE was produced in DF batch culture and prepared for cultivation as described above. For this batch of microalgae cultivation on FW-DFE, the potential effect of ethanol concentration was investigated for *O*. *danica* and *C*. *vulgaris.* Biomass harvesting was followed by *in situ* transesterification and/or esterification in the presence of 15% and 30% v/v ethanol without microbeads addition, and the production of FAEE and FFA during of the reaction course (from 5 minutes to 48 hours) was assessed. In addition, some control experiments were conducted: reaction without ethanol (e.g. ethanol 0%, corresponding to hydrolysis only), reaction with heat treatment prior to incubation for reaction (+HT: 100°C, 10 min inducing denaturation of endogenous enzymes of the microalgae), and reaction with addition of exogenous lipases/acyltransferase (+LIP: rCpLIP2-YS, 200 U/mL) in freshly concentrated biomass. In all conditions the medium was mixed by magnetic stirring, at 30°C during 24h. rCpLIP2-YS is a lipase from the yeast *Candida parapsilosis* obtained as described in the publication of Deniau et al., 2018, Chembiochem, 19(17): 1839-1844.

### O. danica

Cultivation of *O. danica* on FW-DFE led to the production of 2.78 g/L of biomass and complete acetate and butyrate consumption in 10 days. The biomass contained 17.6% of total lipids in the dry matter quantified by MTBE/MeOH extraction and weighing (e.g. 0.49 g of total lipids/L of culture). The results of *in situ* transesterification and/or esterification are shown in figure 10 (concentration of free fatty acids (FFA) and fatty acid ethyl esters (FAEE) in the transesterification and/or esterification reaction medium) and figure 11 (ratios of FFA and FAEE concentrations reported to total fatty acid content [FFA + FAEE]). It is observed that *in situ* transesterification and/or esterification of the concentrated biomass in the presence of 15 % or 30 % v/v ethanol allowed comparable production of FAEE in 24h (0.75 g/L of reaction medium) but with slightly lower FAEE ratio for the lower ethanol concentration (70% vs 80%). In the presence of 30 % v/v ethanol the amount of FAEE continued to increase between 24 and 48h to reach 0.89 g/L (FAEE ratio of 80%) while it remained stable in the presence of 15 % v/v ethanol. In both conditions, about 65% of the final FAEE amount was produced within 30 min of reaction (0.5 h), after which slower production of FAEE continued concomitantly to FFA consumption. It is also important to notice that in the course of the reaction, production of FAEE was superior to FFA decrease, suggesting that FAEE were not only produced by transesterification of complex lipids such as glycerolipids but also by esterification of FFA.

In the absence of ethanol in the reaction medium, more FFA were produced (0.5 g/L in 10 min and 0.67 g/L in 24h) and very low levels of FAEE were quantified (0.06 g/L in 24h of incubation).

Heat treatment prior to incubation for the reaction stabilized FFA levels at 0.4 g/L and prevented FAEE production despite the presence of 15% v/v ethanol. In the presence of exogenous recombinant lipase rCpLIP2-YS, the production of FAEE was slightly lower than with only the endogenous enzymes (0.65 vs 0.75 g/L) so in the conditions tested the addition of this exogenous biocatalyst did not allow higher reaction efficiency.

### C. vulgaris

Cultivation of *C. vulgaris* on FW-DFE led to the production of 2.74 g/L of biomass and complete acetate and butyrate consumption in 10 days. The biomass contained 24.1% of lipids in the dry matter quantified by MTBE/MeOH extraction and weighing (e.g. 0.66 g of lipids/L of culture).

The results of *in situ* transesterification and/or esterification are shown in figure 12 (concentration of free fatty acids (FFA) and fatty acid ethyl esters (FAEE) in the transesterification and/or esterification reaction medium) and figure 13 (ratios of FFA and FAEE concentrations reported to total fatty acid content [FFA + FAEE]). Like with *O. danica* biomass, the increase in ethanol concentration in the transesterification and/or esterification medium allowed a small increase in FAEE proportion and yield. In 48h, FAEE ratios of 70% and 79% were achieved in the presence of 15% and 30% v/v of ethanol respectively, corresponding to 0.59 and 0.79 g/L of reaction medium. About 65% of the final FAEE amount was produced within 3 h and 2 h of reaction in the presence of 15% and 30% v/v ethanol respectively, after which slower production of FAEE continued concomitantly to FFA consumption.

The production of FAEE was also superior to FFA decrease, suggesting again that FAEE were not only produced by transesterification of complex lipids such as glycerolipids but also by esterification of FFA.

In the absence of ethanol, more FFA were produced (0.3 g/L in 10 min and 0.4 g/L in 24h) while very low levels of FAEE were quantified.

Heat treatment prior to incubation stabilized FFA levels at 0.3 g/L and prevented FAEE production despite the presence of 15% v/v ethanol.

In the presence of exogenous recombinant lipase rCpLIP2-YS, the production of FAEE was slightly lower than with only the endogenous enzymes (0.54 vs 0.59 g/L) demonstrating that the addition of this exogenous biocatalyst did not allow higher reaction efficiency in the conditions tested.

### B.3.2/ Cultivation of the microalgae on undiluted effluents from continuous dark fermentation

The *C. vulgaris* and *O. danica* strains were used for this experiment.

For this test, FW-DFE was collected from a dark fermenter already running in continuous conditions as described above and were frozen and stored at -20°C until use. Complete (raw) dark fermentation effluents were thawed overnight at 4°C and used directly for microalgae cultivation without any additions, after adjusting the pH to 7. Microalgae were added at a final OD750nm of 0.1 and cultivated in identical conditions as previously. The pH was manually adjusted to 7 daily.

This FW-DFE contained a very high bacterial biomass concentration which impeded light penetration. However, *O*. *danica* and *C. vulgaris* were still able to grow reaching high amount of dry matter (including bacteria) in stationary phase (8.1 g/L and 6.4 g/L respectively). It was observed that *O. danica* was the most robust microalgae producer on any type of DFE in presence of bacteria by account of its phagotrophic capacities.

Biomass harvesting was followed by *in situ* transesterification and/or esterification in the presence of 20% v/v ethanol or methanol, without microbeads addition, and the reaction medium was mixed by magnetic stirring, at 30°C during 24h. Consequently to high dry matter in the reaction medium (80 and 100 g/L, which included bacteria) the final total fatty acid content was also high: superior to 3 g/L with *O. danica* and close to 5 g/L with *C. vulgaris.* The results of *in situ* transesterification and/or esterification are shown in figure 14 (ratios of FFA and FAEE or FAME concentrations reported to total fatty acid content). It is observed that with *O. danica* concentrated biomass, addition of ethanol or methanol led again to comparable FAEE ratio (63%), with a valuable FAEE concentration of 3.3 g/L in the reaction medium (but also with 1.9 g/L of FFA). These results confirm the high potential of this versatile strain for lipid derivatives production from microalgae cultivated on raw effluent.

Results obtained with *C. vulgaris* concentrated biomass showed that the addition of methanol or ethanol led to a high ester ratio. High levels of fatty esters content were produced in the reaction (1.5 g/L of FAEE with ethanol and 2.2 g/L of FAME with methanol).

### B.3.3/ Cultivation of the microalgae on diluted effluents from continuous dark fermentation

The *C. vulgaris* and *O. danica* strains were used for this experiment.

For this test, the same continuous dark fermentation was used as under section B.3.2/ except this time it was diluted 2-fold, and 7.5 mM NH₄ and 1 mM KPO₄ were added to prevent any growth limitation due to nutrient deficiency. Microalgae were added at a final OD750nm of 0.1 and cultivated in identical conditions as previously. The pH was manually adjusted to 7 daily.

Here, *O. danica* and *C. vulgaris* grew until reaching dry matter concentrations in the stationary phase of 2.5 g/L and 2.4 g/L respectively. Due to the very fast microalgal growth, the microalgae probably used relatively more organic substrate leaving less for bacterial growth. It was observed that in these conditions both *O. danica* and *C. vulgaris* grew optimally, likely due to a more appropriate light penetration and possibly also due to N and P addition. Due to better light penetration, the cultures were probably more oxygenated which stimulated bacterial respiration, translating into fast and complete metabolite consumption.

Biomass harvesting was carried out at early stationary phase and followed by *in situ* transesterification and/or esterification in the presence of 20% v/v ethanol, without microbeads addition, and the reaction medium was mixed by magnetic stirring, at 30°C during 24h.

Here, the final total fatty acid content was 1.6 g/L with *O. danica* and close to 1.5 g/L for *C*. *vulgaris.*

The ratios of FAEE/(FAEE+FAA) obtained in the reaction medium were 75% for *O*. *danica* and 47% for *C*. *vulgaris.*

### B.3.4/ Conclusion about effluents from continuous dark fermentation

With both strains *O. danica* and *C. vulgaris,* high FAEE or FAME ratios were obtained with biomasses cultivated on all types of effluents tested, the best ratios being obtained for effluents from batch dark fermentation mode, or on diluted effluents from continuous dark fermentation.

The profile of fatty acids / esters obtained by the method of the invention implementing 20% v/v ethanol, and undiluted raw continuous dark fermentation effluent from food waste as growth medium are shown by way of example on figure 15 for *O. danica* and figure 16 for *C*. *vulgaris.*

It is first interesting to notice that FFA and FAEE profiles are similar for the two strains but that FFA composition is different to that of FAEE. Indeed, for both strains the major FFA is the C16:0 (14% and 23% for *O*. *danica* and *C. vulgaris* respectively) while the major FAEE is the sum of C18:1 and C18:3 (co-eluted, 29% and 18% respectively). Unsaturated ethyl esters of FA (C16:1, C18:1, C18:2 and C18:3) are the major molecular forms compared to the free acid form (ratio of unsaturated FAEE on unsaturated (FFA+FAEE) of 75% and 65% for *O*. *danica* and *C. vulgaris* respectively).

## Claims

1. A method of producing an ester of a fatty acid and a C₁-C₄ alcohol, **characterized in that** it comprises successive steps of:
- cultivating cells of a microalga strain on a suitable growth medium in suitable conditions to promote cell growth,
- harvesting the cultivated cells and concentrating them to obtain a wet cell pellet,
- suspending said wet cell pellet in an hydroalcoholic solution comprising said C₁-C₄ alcohol,
- incubating the cell suspension thus formed, under stirring, at a temperature between 10 and 60 °C for 30 minutes to 48 hours, so as to form said ester of a fatty acid and said C₁-C₄ alcohol by esterification and/or transesterification of lipids produced by the microalga cells catalyzed by endogenous enzymes of said cells, said incubating step not implementing any exogenous catalyst of esterification and/or transesterification reactions,
- and recovering said ester of a fatty acid and a C₁-C₄ alcohol from said cell suspension.

2. The method as claimed in claim 1, wherein said growth medium comprises at least one of: a C₁-C₆ fatty acid, lactate, ethanol, glucose, glycerol, carbon dioxide.

3. The method as claimed in claim 2, wherein said growth medium comprises acetate and/or butyrate.

4. The method as claimed in any one of claims 1 to 3, wherein said hydroalcoholic solution comprises 15 to 50 % v/v, preferably 15 to 30 % v/v, of C₁-C₄ alcohol(s).

5. The method as claimed in any one of claims 1 to 4, wherein the duration of said incubating step is between 3 and 24 hours.

6. The method as claimed in any one of claims 1 to 5, wherein said microalga strain is a phagotrophic microalga strain.

7. The method as claimed in claim 6, wherein said microalga strain belongs to the genus *Ochromonas*, and preferably to the species *Ochromonas danica.*

8. The method as claimed in any one of claims 1 to 5, wherein said microalga strain belongs to the genus *Chlorella,* and preferably to the species *Chlorella vulgaris* or *Chlorella sorokiniana.*

9. The method as claimed in any one of claims 1 to 8, wherein said step of recovering said ester from said cell suspension is carried out by extraction of said ester from said cell suspension in a non-polar aprotic organic solvent.

10. The method as claimed in any one of claims 1 to 9, wherein said growth medium contains bacteria, in particular a dark fermentation bacterial community.

11. The method as claimed in any one of claims 1 to 10, wherein said growth medium is a synthetic medium.

12. The method as claimed in any one of claims 1 to claim 10, wherein said growth medium is a dark fermentation effluent.

13. The method as claimed in claim 12, wherein said dark fermentation effluent is obtained by the dark fermentation of food waste.

14. The method as claimed in claim 12 or 13, wherein said dark fermentation effluent is not subjected to any purification or sterilization step before being used for said cultivating step.

15. The method as claimed in any one of claims 12 to 14, comprising prior steps of:
- performing dark fermentation of a feedstock, in particular food waste, in anaerobic conditions using a suitable bacterial community,
- collecting an effluent from said dark fermentation,
- and inoculating said effluent with cells of said microalga strain, said effluent then forming said growth medium for said cultivating step.

16. A device for implementing a method as claimed in any one of claims 12 to 15, for producing an ester of a fatty acid and a C₁-C₄ alcohol from a feedstock, **characterized in that** it comprises:
- a dark fermenter,
- a culture reactor configured for performing microalga culture under mixotrophy conditions,
- a system for collecting effluent from said dark fermenter and introducing it into said culture reactor,
- a system for harvesting microalga cells from a culture medium contained in said culture reactor and concentrating them into a wet cell pellet,
- a production reactor equipped with stirring means and heating means,
- and a system for recovering fatty substances from an hydroalcoholic medium contained in said production reactor.
